# NEUE EUROPÄISCHE PATENTSCHRIFT

(11) **EP 1 759 646 B2**
(45) Veröffentlichungstag und Bekanntmachung des Hinweises auf die Entscheidung über den Einspruch: **16.03.2011**
(45) Hinweis auf die Patenterteilung: 30.07.2008
(21) Anmeldenummer: 06018027.0
(22) Anmeldetag: 29.08.2006
(51) Int. Cl.: A61B 17/70

(54) **Kraftschlüssige Schrauben-/Stabverbindung mit durch Verformung induziertem Formschlussanteil**
Non-positive rod/screw connection having positive contribution induced by deformation
Liaison tige/vis par friction et complémentarité de forme induite par déformation

(30) Priorität: 29.08.2005 CH 14092005; 21.11.2005 US 738695 P
(43) Veröffentlichungstag der Anmeldung: 07.03.2007
(73) Patentinhaber: BIRD Biedermann AG, 6072 Sachseln (CH)
(72) Erfinder: Freudiger, Stefan, 3047 Bremgarten (CH)
(74) Vertreter: Hofer, Dorothea

(56) Entgegenhaltungen:
- DE-A1- 4 425 357
- US-A- 0 172 025
- US-A1- 2004 138 660

## Beschreibung

Die vorliegende Erfindung betrifft ein dynamisches Stabilisiersystem für Wirbelsäulen, welches die Wirbelsäule ohne Versteifung zu stabilisieren vermag. Die elastischen Verbindungselemente werden hierbei gemäß Patentanspruch 1 auf eine neuartige Weise mit den Knochen- respektive Pedikelschrauben verbunden.

Gemäß des Standes der Technik gibt es für metallische Stäbe eine Vielzahl von Stab-/Schraubenverbindungen, welche vor allem für Fusionsoperationen (Versteifungen) zur Anwendung gelangen. Elastische Systeme, welche die Wirbelsäulensegmente lediglich stützen und stabilisieren, nicht aber versteifen, sind erst vereinzelt erschienen und dementsprechend sind auch Vorrichtungen für den Zusammenschluss der elastischen Verbindungselemente mit den Pedikelschrauben erst vereinzelt vorhanden.

Grundsätzlich sind Stab-/Schraubenverbindungen, wie sie für metallische Stäbe geeignet sind, nicht automatisch auch für elastische Verbindungselemente geeignet, da zum Beispiel elastische Stäbe aus Kunststoff anderen Gesetzmäßigkeiten unterliegen als vergleichsweise steife Stäbe aus Metall. So können elastische Kunststoff-Stäbe nicht einfach mit Kraftschluss geklemmt werden, da sie in der Regel die Klemmkraft durch Fliessen wieder vermindern können. Es sind also Verbindungskonzepte gesucht, welche nebst einer allfälligen kraftschlüssigen Klemmung auch einen Anteil Formschluss aufweisen, welchen sie zum Beispiel durch lokale elastische oder plastische Deformation zu erzielen vermögen.

Es werden im Folgenden bekannte Stab-/Schraubenverbindungen aufgeführt und ihre Nachteile in Bezug auf die vorliegende Erfindung erläutert.

Die nachstehenden Erfindungen können zwar mit ihren jeweils aufgeführten besonderen Merkmalen in der Kontaktzone einen erhöhten Druck und damit ein elastisches oder gegebenenfalls plastisches Fliessen an der Staboberfläche lokal erzielen, was im Falle eines elastischen Kunststoff Stabes jedoch ungenügend wäre: Die Erfindung gemäß der Patentanmeldung WO 95/01132 (Schläpfer et al.) mithilfe der Kugel in der Klemmschraube; die Erfindung gemäß der Patentanmeldung DE 4234118 A1 (Harms et al.) mithilfe des Randes der hohlen Fixierschraube; die Erfindung gemäß der Patentschrift US 5,005,562 (Cotrel) mithilfe eines zirkulären Zackenprofils an der Klemmschraube; die Erfindung gemäß der Patentanmeldung WO 03/015648 A1 (McKinley) mithilfe von Zähnen unter der hutförmigen Klemmschraube.

Die Erfindung gemäß Patentschrift US 6,117,137 (Halm et al.) weist Rillen an der unteren Stabaufnahme im Schraubenkopf auf, welche jedoch nur einem zusätzlichen Halt gegen Längsverschiebungen dienen. Desweiteren weist die Gegenseite dieser Rillen keine speziell dazu passende Komplementär-Struktur auf.

Die Erfindung gemäß Patentschrift EP 0 689 798 B1 (Sebastian Bueno et al.) weist eine zu einem runden Stab nicht kongruente ("eierförmige") Aufnahme auf, was die Klemmkraft eines Metallstabes zu erhöhen vermag. Da dieses Profil auf der Gegenseite keine speziell dazu passende Form aufweist, ist es wegen Fliess- und Spannungsabfallgefahr für einen elastischen Kunststoff Stab nicht geeignet.

Die Erfindungen gemäß Patentschrift EP 1 364 622 B1 (Freudiger) und Patentanmeldung EP 1 527 742 A1 (Freudiger) weisen gegenseitig geometrisch passende Formschlussverankerungen auf und sind somit für die Verbindung eines elastischen Kunststoff-stabes mit einer Pedikelschraube geeignet. Hingegen verlangt die Positionierung der gerillten Oberflächen ein sehr präzises Einfahren, um ein Verkanten zu vermeiden. Desweiteren lassen Rillenoberflächen kein stufenloses Positionieren zu.

Die Erfindungen gemäß Patentschrift US 6,478,797 B1 (Paul) und Patentanmeldung US 2003/0125742 A1 (Yuan et al.) weisen beide Füllstücke auf, welche von oben eingeschoben ein Rundum-Klemmen des Metall-Stabes erlauben. Keines der beiden Systeme weist jedoch Oberflächenstrukturen im Klemmbereich auf, welche durch einen ausreichenden Formschlussanteil für einen elastischen Kunststoff-Stab geeignet wären.

Die Erfindung gemäß Patentanmeldung FR 2739548 (Huitema) weist eine Rillenverbindung auf einem Teilumfang des Metall-Stabes auf. Da die Hülse mit der Rille schiebend auf den Stab aufgezogen werden muss, ist diese für einen elastischen Kunststoff-Stab, wegen Verklemm-Gefahr nicht geeignet.

DE 44 25 357 A1 beschreibt ein Wirbelsäulenimplantat nach dem Oberbegriff des Patentanspruchs 1.

US 2004/01 38 660 A1 beschreibt einen Verschlusskappenaufbau zum Blockieren eines Wirbelsäulenstabs in einem Aufnahmeteil. An dem inneren Verriegelungselement ist ein deformierbarer Ring vorgesehen, der den Stab kontaktiert und der beim Aufschrauben der Kappe gegen die Staboberfläche gedrückt wird uns sich dabei deformiert. Dies verursacht eine Verbindung auf der Basis eines Kaltverschweißens zwischen dem Stab und dem Verschlusskappenaufbau.

Der vorliegenden Erfindung liegen demnach die Aufgaben zugrunde, einen elastischen Kunststoff-Stab mit kontinuierlicher glatter Oberfläche stufenlos und sicher mit einer Knochen-, respektive Pedikelschraube zu verbinden und dabei sowohl Zug- und Druck- wie auch Scher- und Torsionskräfte zwischen benachbarten Wirbelkörpern zu übertragen.

Die Lösung dieser Aufgabe zeichnet sich dadurch aus, dass die Verbindung eine Kombination aus direktem Kraft- und indirektem Formschluss darstellt. Der indirekte Formschluss wird dabei durch lokale elastische, respektive plastische Deformation (zum Beispiel durch Fliessen) des Kunststoffes erzielt. Idealerweise ist dabei die Ausdehnung des Formschlusses größer als die Einschnürung des elastischen Kunststoff-Stabes unter den zu erwartenden Zugkräften. Idealerweise sind die Volumina von Erhebungen und Vertiefungen ähnlich oder gleich groß, so dass nach Abschluss des Fliessvorganges das Volumen des Kunststoff-Stabes in der Verbindungszone wieder ungefähr den ursprünglichen Wert einnimmt. Idealerweise stellt die Kontaktoberfläche der Schraubenverbindung mit dem Stab einen Käfig dar, welcher ein unkontrolliertes Herausfliessen von Stabmaterial und damit verbunden eine unkontrollierte Lageänderung des Stabes zu vermeiden vermag.

Gegenstand der Erfindung ist demzufolge die im Patentanspruch 1 definierte kraftschlüssige Schrauben-/Stabverbindung mit indirektem Formschlussanteil. Diese ist in der Lage, einen glatten elastischen Kunststoff-Stab derart mit dem Kopf einer Knochen-, respektive Pedikelschraube zu verbinden, dass die zu erwartenden Kräfte, aufgrund der Anwendung als dynamische Stabilisierung der Lendenwirbelsäule, dauerhaft und sicher übertragen werden können. Dabei liegt der Vorteil in der Kombination einer einfachen und stufenlos positionierbaren kraftschlüssigen Verbindung mit der Zuverlässigkeit einer formschlüssigen Verbindung. Der Formschluss wird aber erst bei der Verbindung geschaffen, indem das Fliessverhalten eines elastischen Kunststoffes ausgenutzt wird. Der besondere Vorteil der vorliegenden Erfindung Liegt demnach in der einfachen und sicheren Handhabung des Systems unter Operationsbedingungen.

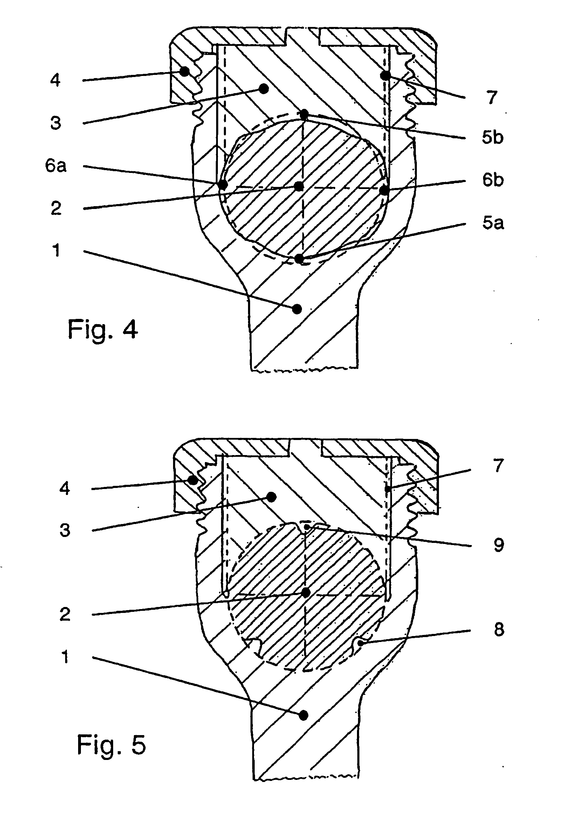

Im Folgenden wird die vorliegende Erfindung anhand der beiliegenden Zeichnungen, welche lediglich Ausführungsbeispiele darstellen, näher erläutert Fig. 5 ist ein Vergleichsbeispiel.

Es zeigen schematisch:
Fig. 1a einen unbelasteten runden und einen belasteten ovalen Querschnitt.
Fig. 1b einen unbelasteten quadratischen und einen belasteten rechteckigen Querschnitt.
Fig. 1c einen unbelasteten dreieckigen und einen belasteten dreieckigen Querschnitt mit verbreiteter Basis.
Fig. 2 ein beispielsweise rundes Verbindungselement (2) in der Aufnahme einer Pedikelschraube (1) mit einem Füllstück (3) und einem Klemmelement (4).
Fig. 3 eine Seitenansicht von Fig. 2.
Fig. 4 einen Schnitt von Fig. 2 mit lokalen Abweichungen vom Querschnitt des Verbindungselementes entlang der Aufnahme (5a, 5b, 6a, 6b).
Fig. 5 die nicht die Erfindung zeigt, zeigt einen Schnitt von Fig. 2 mit zapfenförmigen Abweichungen (8, 9) vom Querschnitt des Verbindungselementes (2) entlang der Aufnahme an der Schraube (1) und am Füllstück (3).
Fig. 6a ein Füllstück (3) mit einer beispielsweisen Aufnahme (10) für ein Gegenhalteinstrument.
Fig. 6b eine Draufsicht von Fig. 6a.
Fig. 6c dieselbe Draufsicht wie Fig. 6b, jedoch mit einer alternativen seitlichen Führung (7).
Fig. 7a eine perspektivische Ansicht eines Füllstücks gemäß einer Abwandlung.
Fig. 7b eine Ansicht des Füllstücks aus Fig. 7a von unten.

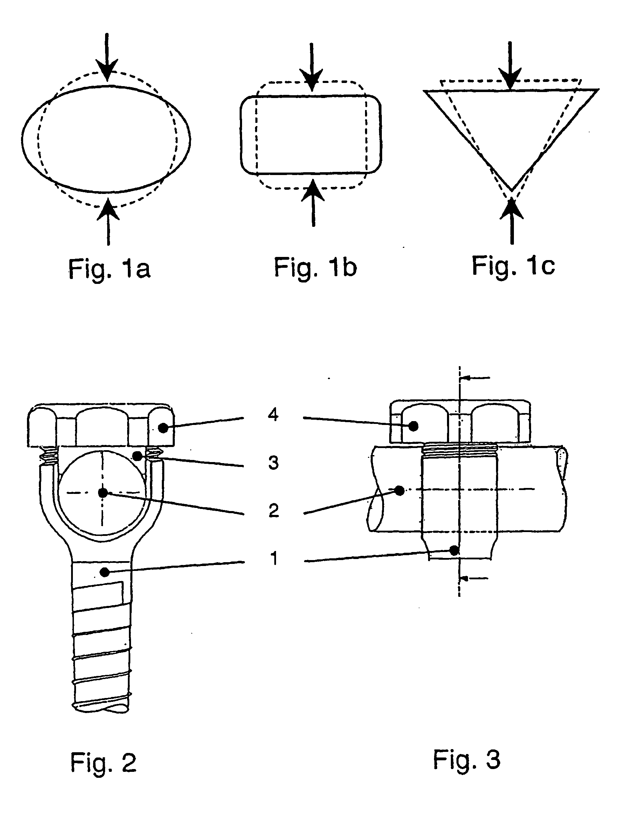

Fig. 1 a stellt zum Beispiel einen runden Querschnitt dar, der durch zwei gegenüberliegende Kräfte in eine neue Form, nämlich einen ovalen Querschnitt übergeführt wird.
Fig. 1b stellt zum Beispiel einen quadratischen Querschnitt dar, der durch zwei gegenüberliegende Kräfte in eine neue Form, nämlich einen rechteckigen Querschnitt übergeführt wird.
Fig. 1c stellt zum Beispiel einen dreieckigen Querschnitt dar, der durch zwei gegenüberliegende Kräfte in eine neue Form, nämlich wiederum ein Dreieck mit spitzeren Basiswinkel übergeführt wird.
Fig. 2 zeigt einen beispielsweisen runden Stab (2) in der Aufnahme einer Pedikelschraube (1), der über ein Füllstück (3) durch beispielsweise eine Mutter (4) als Klemmelement eingespannt wird.
Fig. 3 zeigt dieselben Gegenstände wie Fig. 2, jedoch in einer Seitenansicht.
Fig. 4 zeigt einen Schnitt von Fig. 2 in welchem der Kontaktbereich in einem oder mehreren Schnitte vom Ausgangsquerschnitt des beispielsweisen runden Stabes (2) als beispielsweises Verbindungselement gezielt abweicht, indem zum Beispiel eine erhöhte Rippe unten (5a) an der Schraube (1) und oben (5b) am Füllstück (3) angebracht werden. Hierbei wird das Verbindungselement durch die Mutter (4) als beispielsweises Klemmelement über das beispielsweise seitlich geführte (7) Füllstück (3) zusammengedrückt. Zur Aufnahme des derart verdrängten Materials werden entsprechende Vertiefungen links (6a) und rechts (6b) je an der Schraube (1) und Füllstück (3) angebracht, so dass das verdrängte Material in die Vertiefungen (6a, 6b) fließen kann und somit einen Anteil Formschluss bewirken kann.
Fig. 5 zeigt einen Schnitt von Fig. 2 in welchem die Erhöhungen (5a, 5b) gemäß Fig. 4 zu einem oder mehreren konischen Zapfen (8) an der Schraube (1) respektive einem oder mehreren konischen Zapfen (9) am seitlich geführten (7) Füllstück (3) reduziert werden. Hierbei dringen die konischen Zapfen (8, 9) derart in den elastischen Kunststoff ein, dass ein Anteil Formschluss entsteht.

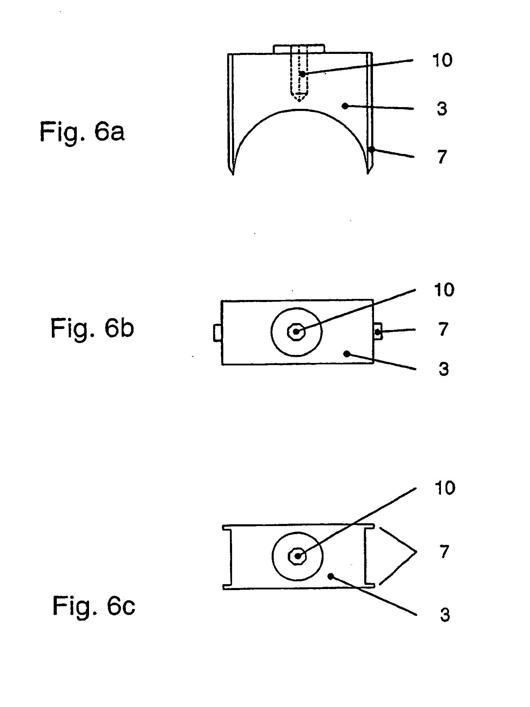

Fig. 6a zeigt eine Seitenansicht eines Füllstückes (3) mit einer beispielsweisen seitlichen Führung (7) und einer beispielsweisen Aufnahme für ein Gegenhalteinstrument (10).
Fig. 6b zeigt eine Draufsicht von Fig. 6a.
Fig. 6c zeigt in einer weiteren Draufsicht von Fig. 6a eine alternative seitliche Führung (7).

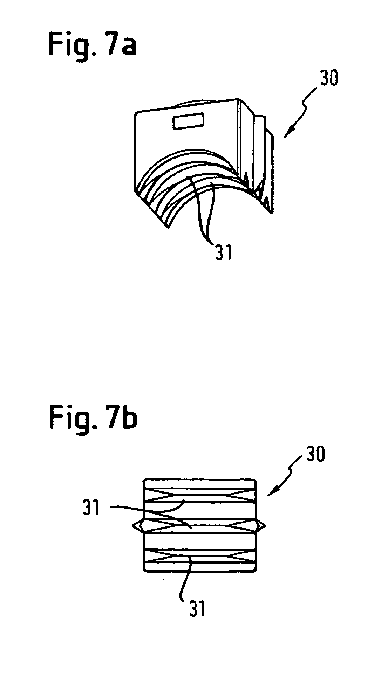

Eine weitere Abwandlung des Füllstücks ist in den Figuren 7a und 7b dargestellt. Das Füllstück 30 unterscheidet sich von dem zuvor beschriebenen Füllstück darin, dass auf der dem Verbindungselement 2 zugewandten Seite mehrere vorspringende Rippen 31 ausgebildet sind, die jeweils senkrecht zur Längsachse des Verbindungselements 2 verlaufen. In dem dargestellten Beispiel sind drei vorspringende Rippen 31 parallel zueinander und gleichmäßig beabstandet ausgebildet. Es können jedoch auch mehr oder weniger Rippen vorhanden sein. Die Abstände können variieren.

Entsprechend zu den Rippen im Füllstück 30 können in der Aufnahme der Schraube 1 ebenfalls mehrere vorspringende Rippen vorgesehen sein, die senkrecht zur Längsachse des Verbindungselements 2 verlaufen. Die Ausbildung von mehreren solchen Rippen führt zu einer verbesserten Lastverteilung an der Oberfläche des Verbindungselements 2.

Die Anzahl der Rippen am Füllstück und in der Aufnahme der Schraube können voneinander verschieden sein.

## Patentansprüche

1. Wirbelsäulenimplantat, bestehend aus einem Verbindungselement (2), mehreren Knochenschranben (1) mit je einer Aufnahme für dieses Verbindungselement, je einem seitlich geführten Füllstück (3) und je einem Klemmelement (4) derart, dass das Verbindungselement (2) zwischen der Aufnahme im Schraubenkopf und dem Füllstück (3) durch das Klemmelement (4) kraftschlüssig geklemmt wird, wobei die Kontur im Bereich der unteren Aufnahme am Schraubenkopf (5a) und im Bereich der oberen Aufnahme am Füllstück (5b) von der Kontur des Verbindungselementes abweicht, wobei das Verbindungselement (2) als elastischer Kunststoffstab mit kontinuierlicher glatter Oberfläche ausgebildet ist,
**dadurch gekennzeichnet, dass** die Kontur auch im Bereich der seitlichen Aufnahmen am Schraubenkopf und Füllstück (6a, 6b) von derjenigen des Verbindungselementes abweicht und wobei die oberen und unteren Abweichungen vorstehend (5a 5b) und die seitlichen Abweichungen vertieft (6a, 6b) sind.

2. Wirbelsäulenimplantat nach Anspruch 1, **dadurch gekennzeichnet, dass** sich die Abweichungen der Kontur in einem oder mehreren Schnitten quer zur Längsache des Verbindungselementes, in derselben Ebene oder in verschiedenen Ebenen befinden.

3. Wirbelsäulenimplantat nach einem der Anspruch 1 oder 2, **dadurch gekennzeichnet, dass** die Abweichungen von der Kontur rippenförmig (5a, 5b, 6a, 6b) oder zapfenförmig (8, 9) sind.

4. Wirbelsäulenimplantat nach einem der Ansprüche 1 bis 3, **dadurch gekennzeichnet, dass** der Stab rund ist.

5. Wirbelsäulenimplantat nach einem der Ansprüche 1 bis 4, **dadurch gekennzeichnet, dass** das Verbindungselement (2) aus PCU (polycarbonateurethane) gefertigt ist.

6. Wirbelsäulenimplantat nach einem der Ansprüche 1 bis 5, **dadurch gekennzeichnet, dass** das Klemmelement (4) eine Mutter ist.

7. Witbelsäutenimplantat nach einem der Ansprüche 1 bis 6, **dadurch gekennzeichnet, dass** das Füllstück (3) eine Aufnahme für ein Halteinstrument (10) aufweist.

## Claims

1. Spinal column implant, comprising a connection element (2), a plurality of bone screws (1), each with a seat for this connection element, each with a laterally guided filling piece (3) and each with a clamping element (4), such that the connection element (2) is clamped with a force fit by the clamping element (4) between the seat in the screw head and the filling piece (3), the contour in the area of the lower seat (5a) on the screw head and in the area of the upper seat (5b) on the filling piece deviating from the contour of the connection element, the connection element (2) being formed as an elastic plastic rod having a continuous smooth surface, **characterized in that** the contour in the area of the lateral seats (6a, 6b) on the screw head and filling piece also deviates from that of the connection element and the upper and lower deviations being prominent (5a, 5b) and the lateral deviations being recessed (6a, 6b).

2. Spinal column implant according to Claim 1, **characterized in that** the deviations of the contour are situated in one or more sections transverse to the longitudinal axis of the connection element, in the same plane or in different planes.

3. Spinal column implant according to either of Claims 1 and 2, **characterized in that** the deviations of the contour are rib-shaped (5a, 5b, 6a, 6b) or peg-shaped (8, 9).

4. Spinal column implant according to one of Claims 1 to 3, **characterized in that** the rod is round.

5. Spinal column implant according to one of Claims 1 to 4, **characterized in that** the connection element (2) is made of PCU (polycarbonate urethane).

6. Spinal column implant according to one of Claims 1 to 5, **characterized in that** the clamping element (4) is a screw nut.

7. Spinal column implant according to one of Claims 1 to 6, **characterized in that** the filling piece (3) has a seat for a holding instrument (10).

## Revendications

1. Implant rachidien, composé d'un élément de liaison (2), d'une pluralité de vis à os (1) ayant chacune un logement pour cet élément de liaison, chacune une pièce de remplissage (3) guidée latéralement, et chacune un élément de serrage (4), de manière que l'élément de liaison (2) soit serré, avec une liaison par interaction de forces, entre le logement ménagé dans la tête de vis et la pièce de remplissage (3), au moyen de l'élément de serrage (4), sachant que le contour, dans la zone du logement inférieur sur la tête de vis (5a) et dans la zone du logement supérieur sur la pièce de remplissage (5b), étant différent du contour de l'élément de liaison, l'élément de liaison (2) étant réalisé sous forme de barre en matière synthétique élastique à surface lisse continue,
**caractérisé en ce que**,
également dans la zone des logements latéraux sur la tête de vis et la pièce de remplissage (6a, 6b), le contour est également différent de celui de l'élément de liaison, et les écarts supérieur et inférieur sont en saillie (5a, 5b) et les écarts latéraux sont en creux (6a, 6b).

2. Implant rachidien selon la revendication 1, **caractérisé en ce que** les écarts du contour se trouvent en une ou plusieurs coupes transversalement à l'axe longitudinal de l'élément de liaison, dans le même plan ou dans des plans différents.

3. Implant rachidien selon l'une des revendications 1 ou 2, **caractérisé en ce que** les écarts du contour sont en forme de nervure (4a, 4b, 6a, 6b) ou en forme de tétons (8, 9).

4. Implant rachidien selon l'une des revendications 1 à 3, **caractérisé en ce que** la barre est ronde.

5. Implant rachidien selon l'une des revendications 1 à 4, **caractérisé en ce que** l'élément de liaison (2) est fabriqué en PCU (polycarbonate-uréthane).

6. Implant rachidien selon l'une des revendications 1 à 5, **caractérisé en ce que** l'élément de serrage (4) est un écrou.

7. Implant rachidien selon l'une des revendications 1 à 6, **caractérisé en ce que** la pièce de remplissage (3) présente un logement pour un instrument de maintien (10).
